(19) Europäisches Patentamt
European Patent Office
Office européen des brevets

(11) Numéro de publication: **0 022 017**
**B1**

# (12) FASCICULE DE BREVET EUROPEEN

(45) Date de publication du fascicule de brevet: **05.05.82**

(51) Int. Cl.³: **C 07 C 103/50**, C 07 D 295/14, A 61 K 31/165

(21) Numéro de dépôt: **80400942.1**

(22) Date de dépôt: **24.06.80**

(54) **Nouveaux dérivés de benzoyl-2 nitro-4 anilides, leur préparation et leur application en tant que médicaments.**

(30) Priorité: **25.06.79 FR 7916213**

(43) Date de publication de la demande: **07.01.81 Bulletin 81/1**

(45) Mention de la délivrance du brevet: **05.05.82 Bulletin 82/18**

(84) Etats contractants désignés: **AT BE CH DE GB IT LI LU NL SE**

(56) Documents cités:
**EP-A-0 000 299**
**FR-A-1 462 887**
**FR-A-2 012 022**
**FR-A-2 184 739**
**US-A-3 799 928**

(73) Titulaire: **PIERRE FABRE S.A., 125, rue de la Faisanderie, F-75116 Paris (FR)**

(72) Inventeur: **Mouzin, Gilbert, 21, rue Sainte-Foy, F-81100 Castres (FR)**
Inventeur: **Cousse, Henri, La Foun de Los Nobios Chemin de Lastinos, F-81100 Castres (FR)**

(74) Mandataire: **Martin, Jean-Jacques et al, Cabinet REGIMBEAU 26, Avenue Kléber, F-75116 Paris (FR)**

EP 0 022 017 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# Nouveaux dérivés de benzoyl-2 nitro-4 anilides, leur préparation et leur application en tant que médicaments

La présente invention réalisée au Centre de Recherches Pierre FABRE concerne de nouveaux composés chimiques dérivés de benzoyl-2 nitro-4 anilides, leur procédé de préparation et leur application en thérapeutique.

La technique antérieure par exemple illustrée par la demande de brevet français no 2 012 022, le brevet français no 1 462 887, le brevet américain no 3 799 928, décrit des intermédiaires de synthèse, généralement des composés quadricycliques, qui ne sont doués d'aucune activité pharmacologique propre. Cette absence d'activité des composés de la technique antérieure résulte précisément des différences de structure existant entre ces composés et les nouveaux dérivés selon la présente invention.

En outre la technique antérieure, par exemple illustrée par la demande de brevet français no 2 184 739 et la demande de brevet européen no 0 000 229, concerne également des composés dont la structure et l'activité pharmacologique se différencient de celles des dérivés, objet de la présente invention.

Les composés chimiques, objet de la présente invention, répondent à la formule générale I:

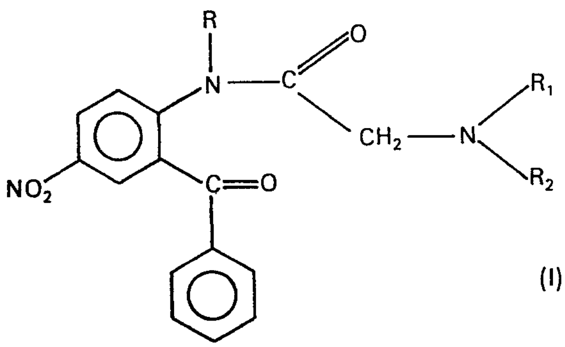

(I)

dans laquelle:
R représente un groupe alcoyle linéaire ou ramifié, un groupe alcényle, cycloalcoyle, ou benzyle;
$R_1$ représente un atome d'hydrogène, un groupe alcoyle ou hydroxyalcoyle;
$R_2$ représente un groupe hydroxyalcoyle, alcényle ou alcynyle éventuellement substitués une ou plusieurs fois par un radical alcoyle, ou un groupe cycloalcoyle de 3 à 6 atomes de carbone;
$R_1$ et $R_2$ peuvent, en outre, former avec l'atome d'azote auquel ils sont reliés un hétérocycle azoté comprenant éventuellement un second hétéroatome choisi parmi l'oxygène et l'azote,
à la condition toutefois que, lorsque $R_2$ représente un groupe hydroxyalcoyle, $R_1$ ne peut être un atome d'hydrogène.

La présente invention s'applique également aux sels des composés de formule (I) obtenus avec des acides minéraux ou organiques thérapeutiquement acceptables.

La présente invention concerne également un procédé de préparation de composés de formule I, caractérisé en ce que l'on condense un composé de formule générale II:

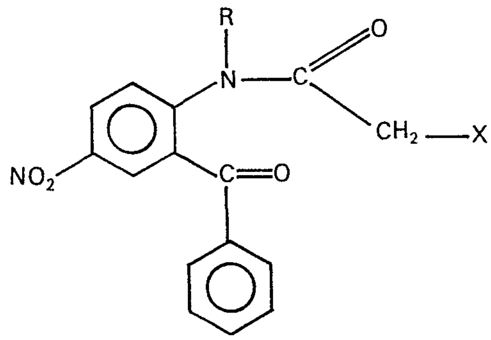

(II)

dans laquelle:
R a la signification donnée à propos de la formule générale (I) et
X représente un atome d'halogène;
avec une amine de formule générale III:

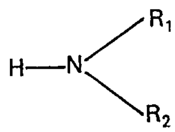

(III)

dans laquelle:
$R_1$ et $R_2$ ont la signification donnée à propos de la formule générale I.

Les produits de formule générale II peuvent être préparés selon l'un des deux procédés suivants:

Procédé a: à partir d'halogéno-2 nitro-5 benzophénone

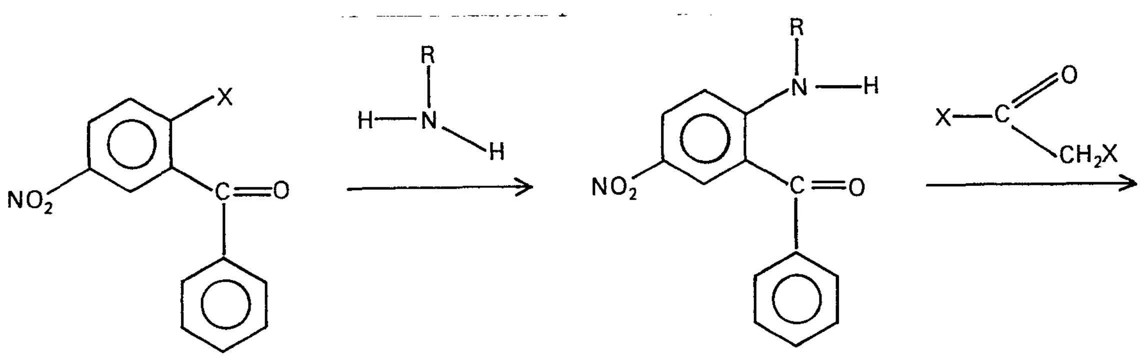

(II)

Procédé b: à partir d'amino-2 nitro-5 benzophénone

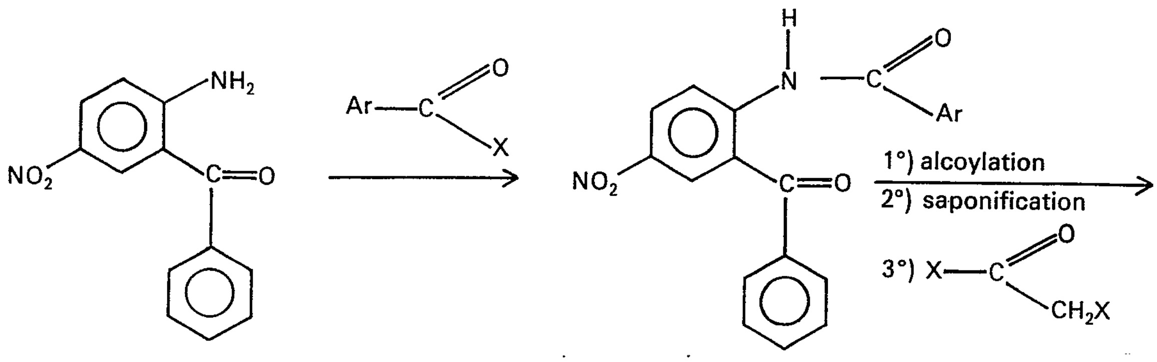


(II)

X et R ayant les définitions données précédemment.

La présente invention concerne enfin l'application des composés de formule générale I en tant que médicaments doués d'une activité sur le système nerveux central, et en particulier en tant que médicaments hypnotiques.

La présente invention sera décrite ci-après plus en détail à propos des exemples non limitatifs suivants:

Exemple 1

Chlorhydrate du N-méthyl benzoyl-2′ nitro-4′ morpholino-2 acétanilide.

A) Préparation de la méthylamino-2 nitro-5 benzophénone

Procédé a: A une suspension de 2,61 g (0,01 mole) de chloro-2 nitro-5 benzophénone dans 20 cc d'éthanol à 95° GL, on ajoute 7,75 cc (0,1 mole) de méthylamine à 40% dans l'eau, puis 0,15 g de chlorure cuivreux et 0,15 g de poudre de cuivre. Après 5 heures de chauffage à 60°C, on évapore jusqu'à siccité et l'on reprend le résidu par une solution d'acide chlorhydrique N.

On extrait à l'acétate d'éthyle, on lave à l'eau jusqu'à neutralité et l'on sèche sur sulfate de sodium. On obtient après recristallisation dans un mélange acétate d'éthyle – hexane (75/25) 2,28 g de cristaux jaunes. Point de fusion 168°C. Rendement environ 90%. Chromatographie sur plaque:

- support: gel de silice 60 F 254 Merck
- solvant: acétate d'éthyle – éther de pétrole 30/70
- révélation: UV et iode
- Rf: 0,73.

Procédé b: On chauffe à 60°C une suspension de 11,07 g (0,031 mole) de benzamido-2 nitro-5 benzophénone et 724 mg (0,003 mole) de chlorure de benzyl triéthylammonium dans 450 cc de toluène. On additionne goutte à goutte simultanément 16 cm³ de soude 1 ON (0,16 mole) et 10 cm³ de sulfate de méthyle (0,1 mole) dans 50 cm³ de toluène. On maintient le mélange réactionnel sous forte agitation à 60°C pendant 8 heures. Après retour à température ambiante, on dilue et l'on décante. On évapore la phase organique et on reprend le résidu par 200 cm³ d'éthanol et 60 cm³ de soude 6N (0,36 mole). Après 2 heures de chauffage à 70°C, on évapore l'éthanol, on reprend le résidu par l'acétate d'éthyle, on lave à l'eau et l'on sèche sur sulfate de sodium. On récupère après recristallisation dans un mélange acétate d'éthyle – hexane (75/25) 7,22 g de cristaux jaunes. Point de fusion 168°C. Rendement 88%.

B) Préparation du N-méthyl benzoyl-2′ nitro-4′ bromo-2 acétanilide

A une suspension de 2,56 g (0,01 mole) de méthylamino-2 benzophénone dans 100 cc de toluène, on ajoute 1,05 cc (0,012 mole) de chlorure de bromacétyle et on chauffe le mélange réactionnel 8 heures à 100°C.

On laisse revenir à température ambiante, puis on additionne une solution glacée de 5 g de carbonate de sodium dans 100 cc d'eau et on agite 15 minutes. On décante, on lave la phase organique à l'eau jusqu'à neutralité, et l'on sèche sur sulfate de sodium. On filtre et l'on évapore la phase organique, l'huile résiduelle obtenue cristallise par trituration dans l'éther. On récupère 2,85 g de cristaux beiges. Point de fusion 95°C. Rendement: 76%.

Chromatographie sur plaque:

- support: gel de silice 60 F 254 Merck
- solvant: acétate d'éthyle – éther de pétrole 30/70
- révélation: UV et iode
- Rf: 0,44

C) Préparation du chlorhydrate de N-méthyl benzoyl-2′ nitro-4′ morpholino-2 acétanilide

A une solution de 3,77 g (0,01 mole de N-méthyl benzoyl-2′ nitro-4′ bromo-2 acétanilide dans 30 cc de chlorure de méthylène, on ajoute rapidement 2,6 cc (0,03 mole) de morpholine. On maintient le mélange réactionnel sous forte agitation pendant 4 heures à température ambiante, puis 1 heure au reflux. On extrait deux fois à l'acide chlorhydrique 3N, les phases agueuses sont traitées au bicarbonate de sodium puis, on extrait à l'acétate d'éthyle.

On lave à l'eau jusqu'à neutralité et l'on sèche sur sulfate de sodium. On décolore par du noir animal. Après filtration et évaporation du solvant, on obtient une huile résiduelle qui est traitée par une solution éthanolique saturée d'acide chlorhydrique.

Le chlorhydrate formé est précipité par addition d'éther et par recristallisation dans un mélange méthanol-éther isopropylique. On récupère avec un rendement de 82% le produit de formule:

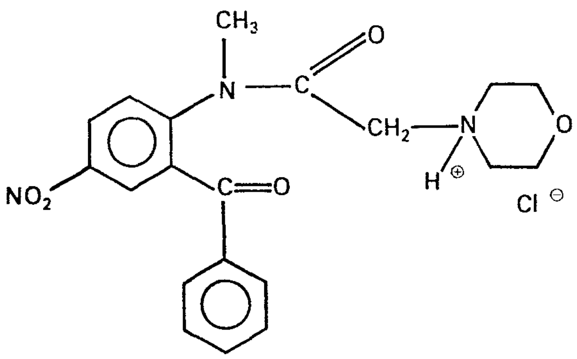

Formule brute: $C_{20}H_{22}ClN_3O_5$

Masse moléculaire: 419,87
Cristaux: blanc cassé
Point de fusion: 170°C
Spectre IR (KBr) ν $cm^{-1}$: 1679-1655 et 1612 Chromatographie sur plaque:

- support: gel de silice 60 F 254 Merck
- solvant: méthanol-chloroforme 15/85
- révélation: UV et iode
- Rf: 0,68

Solubilités: Insoluble dans l'eau. Soluble à 2,5% dans le DMSO et à 10% dans la méthylpyrrolidone.

Exemple 2

Maléate acide de N-éthyl benzoyl-2′ nitro-4′ morpholino-2 acétanilide.

A) Préparation de l'éthylamino-2 nitro-5 benzophénone

D'une façon similaire à celle décrite dans l'exemple 1, A), mais en utilisant l'éthylamine, on obtient l'éthylamino-2 nitro-5 benzophénone avec un rendement de 90%. Point de fusion: 135°C. Cristaux jaunes.

Chromatographie sur plaque:

- support: gel de silice 60 F 254 Merck
- solvant: acétate d'éthyle-éther de pétrole 10/90
- révélation: UV et iode
- Rf: 0,30

B) Préparation du N-éthyl benzoyl-2′ nitro-4′ bromo-2 acétanilide

D'une façon similaire à celle décrite dans l'exemple 1, B) mais en utilisant l'éthylamino-2 nitro-5 benzophénone, on récupère avec un rendement de 95% le N-éthyl benzoyl-2′ nitro-4′ bromo-2 acétonilide. Cristaux blancs. Point de fusion: 95°C.

Chromatographie sur plaque:

- support: gel de silice 60 F 254 Merck
- solvant: acétate d'éthyle-éther de pétrole 30/70
- révélation: UV et iode
- Rf: 0,40

C) Préparation du maléate acide de N-éthyl benzoyl-2′ nitro-4′ morpholino-2 acétanilide

D'une façon similaire à celle décrite dans l'exemple 1, C) mais en utilisant le N-éthyl benzoyl-2′ nitro-4′ bromo-2 acétanilide et l'acide maléique comme agent salifiant, on obtient avec un rendement de 87% le produit de formule:

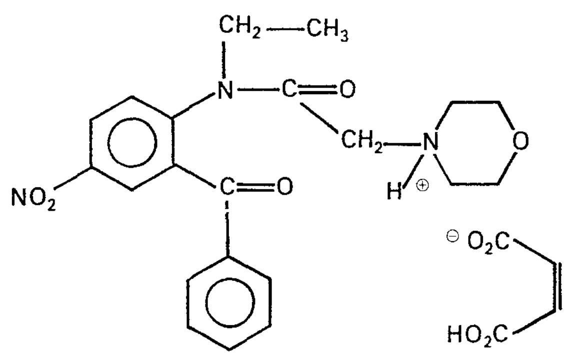

Formule brute: $C_{25}H_{27}N_3O_9$

Masse moléculaire: 513,49
Cristaux blancs
Point de fusion: 165°C

Chromatographie sur plaque:

- support: gel de silice 60 F 254 Merck
- solvant: acétate d'éthyle
- révélation UV et iode
- Rf: 0,24

Exemple 3

Maléate acide de N-propyl benzoyl-2′ nitro-4′ morpholino-2 acétanilide.

A) Préparation de la propylamino-2 nitro-5 benzophénone

D'une façon similaire à celle décrite dans l'exemple 1, A) mais en utilisant la propylamine, on obtient la propylamino-2 nitro-5 benzophénone avec un rendement de 85%.
Point de fusion: 150°C. Cristaux jaunes.

Chromatographie sur plaque:

- support: gel de silice 60 F 254 Merck
- solvant: acétate d'éthyle-éther de pétrole 10/90
- révélation: UV et iode
- Rf: 0,4.

B) Préparation du N-propyl benzoyl-2′ nitro-4′ bromo-2 acétanilide

D'une façon similaire à celle décrite dans l'exemple 1, B) mais en utilisant la N-propylamino-2 nitro-5 benzophénone, on obtient avec un rendement de 80% le N-propyl benzoyl-2′ nitro-4′ bromo-2 acétanilide. Cristaux jaune pâle. Point de fusion: 122°C.

Chromatographie sur plaque:

- support: gel de silice 60 F 254 Merck
- solvant: acétate d'éthyle-éther de pétrole 30/70
- révélation: UV et iode
- Rf: 0,50.

C) Préparation du maléate acide de N-propyl benzoyl-2′ nitro-4′ morpholino-2 acétanilide

D'une façon similaire à celle décrite dans l'exemple 1, C) mais en utilisant le N-propyl benzoyl-2′ nitro-4′ bromo-2 acétanilide et l'acide maléique comme agent salifiant, on obtient avec un rendement de 75% le produit de formule:

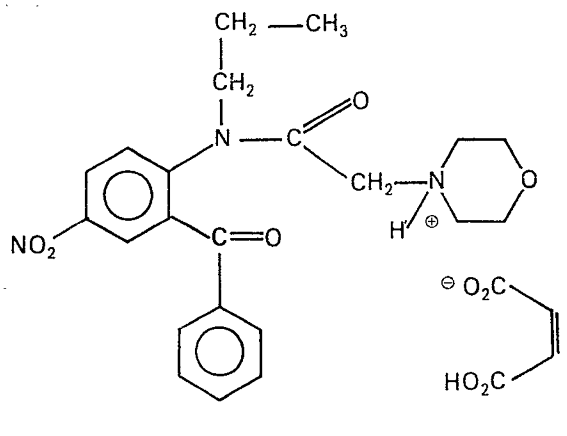

Formule brute: $C_{26}H_{29}N_3O_9$

Masse moléculaire: 527,54
Cristaux: blancs

Point de fusion: 181°C

Chromatographie sur plaque:

- support: gel de silice 60 F 254 Merck
- solvant: acétate d'éthyle
- révélation: UV et iode
- Rf: 0,23

Exemple 4

Maléate acide de N-isopropyl benzoyl-2′ nitro-4′ morpholino-2 acétanilide.

A) Préparation de l'isopropylamino-2 nitro-5 benzophénone

D'une façon similaire à celle décrite dans l'exemple 1, A) mais en utilisant l'isopropylamine, on obtient avec un rendement de 94% l'isopropylamino-2 nitro-5 benzophénone.
Point de fusion: 155°C. Cristaux jaunes.

Chromatographie sur plaque:

- support: gel de silice 60 F 254 Merck
- solvant: acétate d'éthyle-éther de pétrole 10/90
- révélation: UV et iode
- Rf: 0,18

B) Préparation du N-isopropyl benzoyl-2′ nitro-4′ bromo 2 acétanilide

D'une façon similaire à celle décrite dans l'exemple 1, B) mais en utilisant l'isopropylamino-2 nitro-5 benzophénone on obtient avec un rendement de 95% le N-isopropyl benzoyl-2′ nitro-4′ bromo-2 acétanilide. Huile jaune pâle.

Chromatographie sur plaque:

- support: gel de silice 60 F 254 Merck
- solvant: acétate d'éthyle-éther de pétrole 30/70
- révélation: UV et iode
- Rf: 0,50

C) Préparation du N-isopropyl benzoyl-2′ nitro-4′ morpholino-2 acétanilide

D'une façon similaire à celle décrite dans l'exemple 1, C) mais en utilisant le N-isopropyl benzoyl-2′ nitro-4′ bromo-2 acétanilide et l'acide maléique comme agent salifiant, on obtient avec un rendement de 85% le produit de formule:

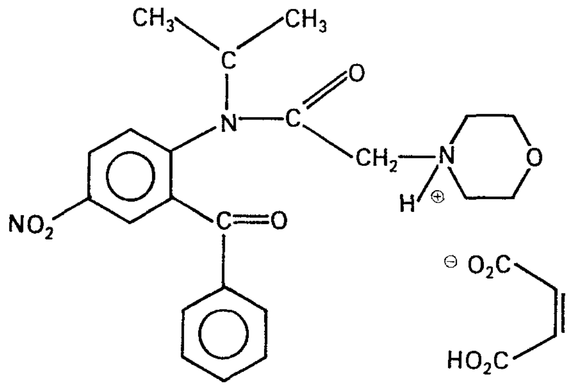

Formule brute: $C_{26}H_{29}N_3O_9$

Masse moléculaire: 527,54
Cristaux blancs
Point de fusion: 183°C

Chromatographie sur plaque:

- support: gel de silice 60 F 254 Merck
- solvant: acétate d'éthyle
- révélation: UV et iode
- Rf: 0,32

Exemple 5
Maléate de N-allyl benzoyl-2′ nitro-4′ morpholino-2 acétanilide

A) Préparation de l'allylamino-2 nitro-5 benzophénone
D'une façon similaire à celle décrite dans l'exemple 1, A) mais en utilisant l'allylamine, on obtient avec un rendement de 95% l'allylamino-2 nitro-5 benzophénone. Point de fusion: 97°C. Cristaux jaunes.

Chromatographie sur plaque:

- support: gel de silice 60 F 254 Merck
- solvant: acétate d'éthyle-éther de pétrole 10/90
- révélation: UV et iode
- Rf: 0,20

B) Préparation du N-allyl benzoyl-2′ nitro-4′ bromo-2 acétanilide
D'une façon similaire à celle décrite dans l'exemple 1, B) mais en utilisant l'allylamino-2 nitro-5 benzophénone, on obtient avec un rendement de 91% le N-allyl benzoyl-2′ nitro-4′ bromo-2 acétanilide.

Huile jaune.

Chromatographie sur plaque:

- support: gel de silice 60 F 254 Merck
- solvant: acétate d'éthyle-éther de pétrole 30/70
- révélation: UV et iode
- Rf: 0,50.

C) Préparation du maléate acide de N-allyl benzoyl-2′ nitro-4′ morpholino-2 acétanilide
D'une façon similaire à celle décrite dans l'exemple 1, C) mais en utilisant le N-allyl benzoyl-2′ nitro-4′ bromo-2 acétanilide et l'acide maléique comme agent salifiant, on obtient avec un rendement de 75% le produit de formule:

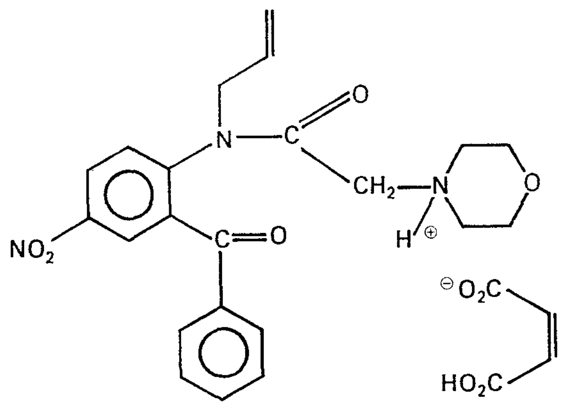

Formule brute: $C_{26}H_{27}N_3O_9$

Masse moléculaire: 525,52
Cristaux blancs
Point de fusion: 165°C.

Chromatographie sur plaque:

- support: gel de silice 60 F 254 Merck
- solvant: acétate d'éthyle
- révélation UV et iode
- Rf: 0,34

Exemple 6
N-benzyl benzoyl-2′ nitro-4′ morpholino-2 acétanilide
A) Préparation de la benzylamino-2 nitro-5 benzophénone
D'une façon similaire à celle décrite dans l'exemple 1, A) mais en utilisant la benzylamine, on obtient avec un rendement de 70% la benzylamino-2 nitro-5 benzophénone.
Point de fusion: 120°C. Cristaux jaunes.

Chromatographie sur plaque:

- support: gel de silice 60 F 254 Merck
- solvant: acétate d'éthyle-éther de pétrole 10/90
- révélation: UV et iode
- Rf: 0,22

B) Préparation du N-benzyl benzoyl-2' nitro-4' bromo-2 acétanilide

D'une façon similaire à celle décrite dans l'exemple 1, B) mais en utilisant la benzylamino-2 nitro-5 benzophénone, on obtient avec un rendement de 94% le N-benzyl benzoyl-2' nitro-4' bromo-2 acétanilide.
Point de fusion: 149°C.
Cristaux blancs.

Chromatographie sur plaque:

- support: gel de silice 60 F 254 Merck
- solvant: acétate d'éthyle-éther de pétrole 30/70
- révélation: UV et iode
- Rf: 0,62

C) Préparation du N-benzyl benzoyl-2' nitro-4' morpholino-2 acétanilide

D'une façon similaire à celle décrite dans l'exemple 1, C) mais en utilisant le N-benzylbenzoyl-2' nitro-4' bromo-2 acétanilide, on obtient avec un rendement de 84% le produit de formule

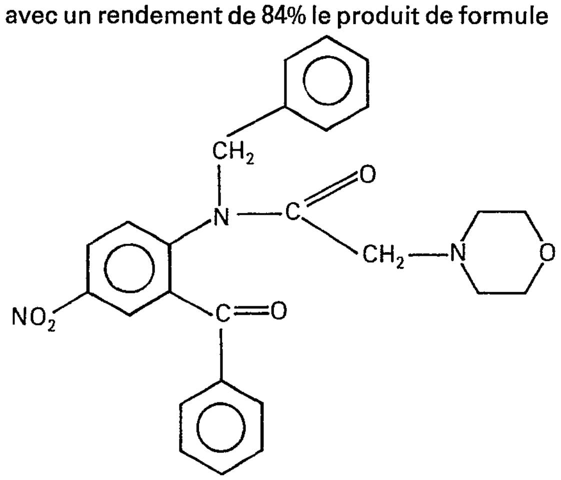

Formule brute: $C_{26}H_{25}N_3O_5$

Masse moléculaire: 459,58
Cristaux blanc cassé
Point de fusion: 110°C

Chromatographie sur plaque:

- support: gel de silice 60 F 254 Merck
- solvant: acétate d'éthyle
- révélation: UV et iode
- Rf: 0,40

Exemple 7

N-cyclopropyl benzoyl-2' nitro-4' morpholino-2 acétanilide

A) Préparation de la cyclopropylamino-2 nitro-5 benzophénone

D'une façon similaire à celle décrite dans l'exemple 1, A) mais en utilisant la cyclopropylamine, on obtient avec un rendement de 65% la cyclopropylamino-2 nitro-5 benzophénone. Cristaux jaunes. Point de fusion: 125°C.

Chromatographie sur plaque:

- support: gel de silice 60 F 254 Merck
- solvant: acétate d'éthyle-éther de pétrole 30/70
- révélation: UV et iode
- Rf: 0,70.

B) Préparation du N-cyclopropylbenzoyl-2' nitro-4' morpholino-2 acétanilide

D'une manière similaire à celle décrite dans l'exemple 1, B) et 1, C) mais en utilisant la cyclopropylamino-2 nitro-5 benzophénone, puis le N-cyclopropyl benzoyl-2' nitro-4' bromo-2 acétanilide, on obtient le produit de formule:

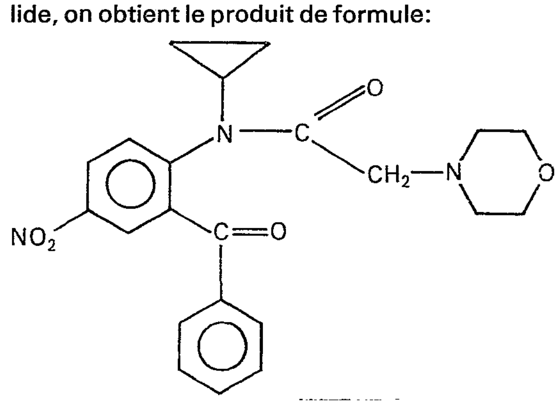

Exemple 8

N-cyclopentyl benzoyl-2' nitro-4' morpholino-2 acétanilide

A) Préparation de la cyclopentylamino-2 nitro-5 benzophénone

D'une façon similaire à celle décrite dans l'exemple 1, A) mais en utilisant la cyclopentylamine, on obtient avec un rendement de 82% la cyclopentylamino-2 nitro-5 benzophénone. Cristaux jaunes. Point de fusion: 95°C.

Chromatographie sur plaque:

- support: gel de silice 60 F 254 Merck
- solvant: acétate d'éthyle-éther de pétrole 30/70
- révélation: UV et iode
- Rf: 0,62

B) Préparation du N-cyclopentylbenzoyl-2′ nitro-4′ morpholino-2 acétanilide

D'une façon similaire à celle décrite dans l'exemple 1, B) et 1, C) mais en utilisant la cyclopentylamino-2 nitro-5 benzophénone et le N-cyclopentylbenzoyl-2′ nitro-4′ bromo-2 acétanilide, on obtient le produit de formule:

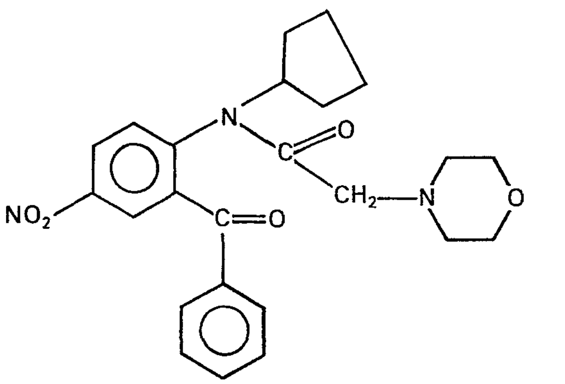

Exemple 9

N-cyclohexylbenzoyl-2′ nitro-4′ morpholino-2 acétanilide

A) Préparation de la cyclohexylamino-2 nitro-5 benzophénone

D'une façon similaire à celle décrite dans l'exemple 1, A) mais en utilisant la cyclohexylamine, on obtient avec un rendement de 80% la cyclohexylamino-2 nitro-5 benzophénone. Cristaux jaunes. Point de fusion: 75°C.

Chromatographie sur plaque:

- support: gel de silice 60 F 254 Merck
- solvant: acétate d'éthyle-éther de pétrole 30/70
- révélation: UV et iode
- Rf: 0,63

B) Préparation du N-cyclohexylbenzoyl-2′ nitro-4′ morpholino-2 acétanilide

D'une façon similaire à celle décrite dans l'exemple 1, B) et 1, C) mais en utilisant la cyclohexylamino-2 nitro-5 benzophénone et le N-cyclohexyl benzoyl-2′ nitro-4′ bromo-2 acétanilide, on obtient le produit de formule:

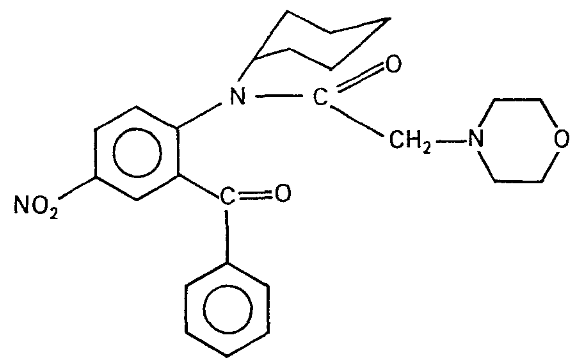

Exemple 10

Chlorhydrate de N-cyclohexyl N′ méthyl benzoyl-2′ nitro-4′ glycylanilide

A une solution de 3,77 g (0,01 mole) de N-méthyl benzoyl-2′ nitro-4′ bromo-2 acétanilide dans 40 cc de chlorure de méthylène on ajoute 2,4 cc (0,021 mole) de cyclohexylamine et l'on chauffe 1 heure à reflux. On évapore la phase organique jusqu'à siccité et l'on reprend le résidu par l'acétate d'éthyle, puis on extrait 3 fois avec 50 cc d'acide chlorhydrique 6N.

On réunit les phases aqueuses qui sont traitées par du bicarbonate de sodium et l'on extrait à l'acétate d'éthyle. On décante, lave à l'eau et sèche sur sulfate de sodium.

Après filtration et évaporation de l'acétate d'éthyle, on récupère une huile que l'on traite par une solution éthanolique saturée d'acide chlorhydrique. Après recristallisation dans l'éthanol, on récupère avec un rendement de 70% le produit de formule:

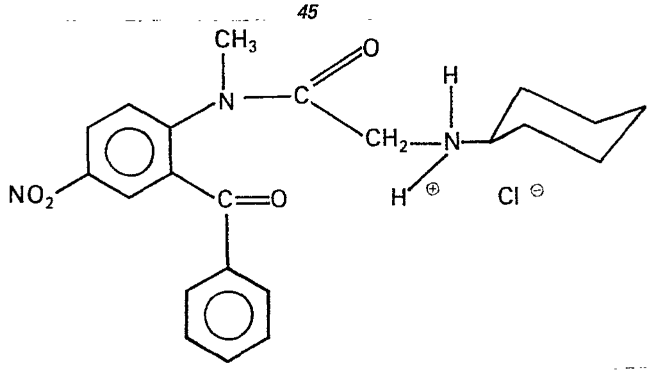

Formule brute: $C_{22}H_{26}ClN_3O_4$

Masse moléculaire: 431,9
Cristaux: blancs
Point de fusion: 204°C

Chromatographie sur plaque:

- support: gel de silice 60 F 254 Merck
- solvant: butanol - acide acétique - eau 6/2/2
- révélation: UV et iode
- Rf: 0,43

Exemple 11

Fumarate de N-N′ diméthyl N-cyclohexylbenzoyl-2′ nitro-4′ glycylanilide

D'une façon similaire à celle décrite dans l'exemple 10 mais en utilisant la N-méthyl cyclohexylamine et l'acide fumarique comme agent salifiant, on obtient avec un rendement de 65% le produit de formule:

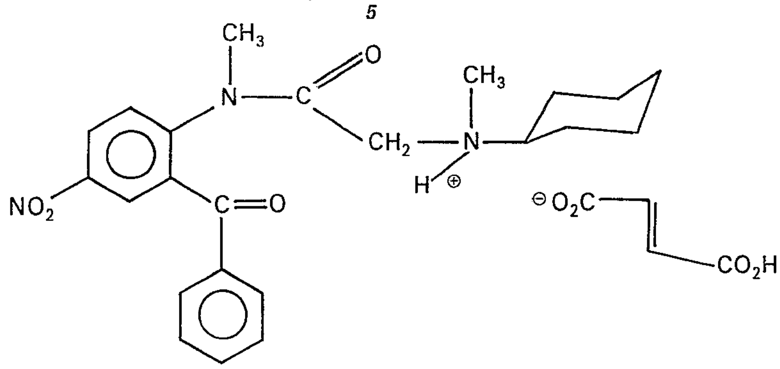

Formule brute: $C_{27}H_{31}N_3O_8$

Masse moléculaire: 525,56
Cristaux: blancs
Point de fusion: 173°C

Chromatographie sur plaque:

- support: gel de silice 60 F 254 Merck
- solvant: butanol – acide acétique – eau 6/2/2
- révélation: UV et iode
- Rf: 0,39

Exemple 12

N′-méthyl N-cyclopropyl benzoyl-2′ nitro-4′ glycylanilide

D'une manière analogue à celle décrit dans l'exemple 10, mais en utilisant la cyclopropylamine, on obtient le produit de formule:

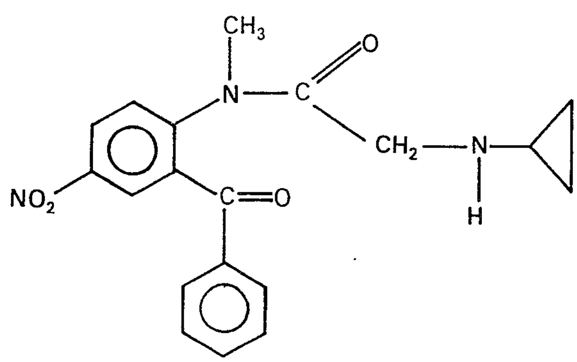

Exemple 13

N′-méthyl N-diméthyl-1-1 propargyl benzoyl-2′ nitro-4′ glycylanilide

D'une manière analogue à celle décrite dans l'exemple 10, mais en utilisant la diméthyl-1-1 propargylamine, on obtient le produit de formule:

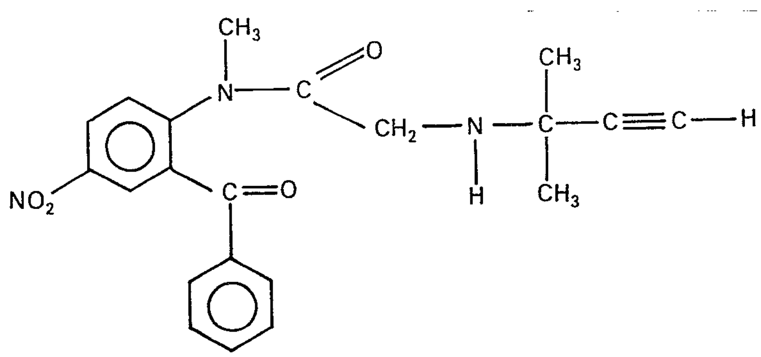

Exemple 14

N′ méthyl N-allyl benzoyl-2′ nitro-4′ glycylanilide

D'une manière analogue à celle décrite dans l'exemple 10, mais en utilisant l'allylamine, on obtient le produit de formule:

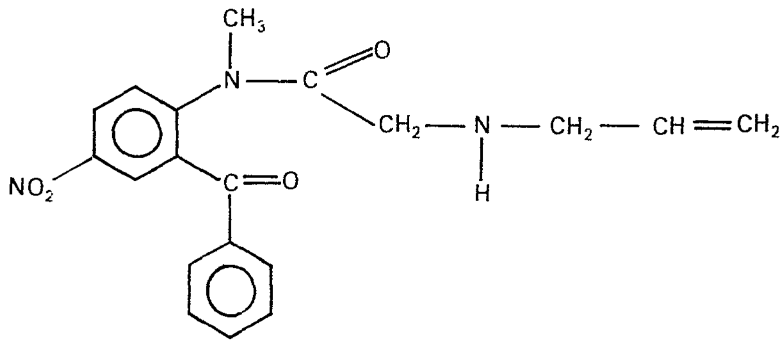

Exemple 15

N-N′ diméthyl N (hydroxy-2 éthyl) benzoyl-2′ nitro-4′ glycylanilide

D'une manière analogue à celle décrite dans l'exemple 10, mais en utilisant la N-méthyl éthanolamine, on obtient le produit de formule:

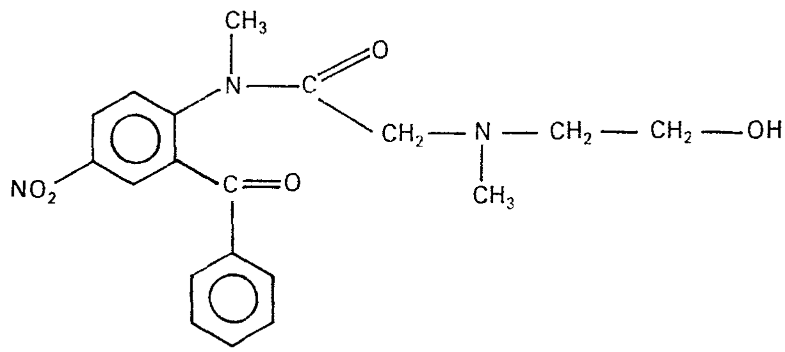

Exemple 16

N′ méthyl N-N-bis (hydroxy-2 éthyl) benzoyl-2′ nitro-4′ glycylanilide

D'une façon analogue à celle décrite dans l'exemple 10, mais en utilisant la diéthanolamine, on obtient le produit de formule:

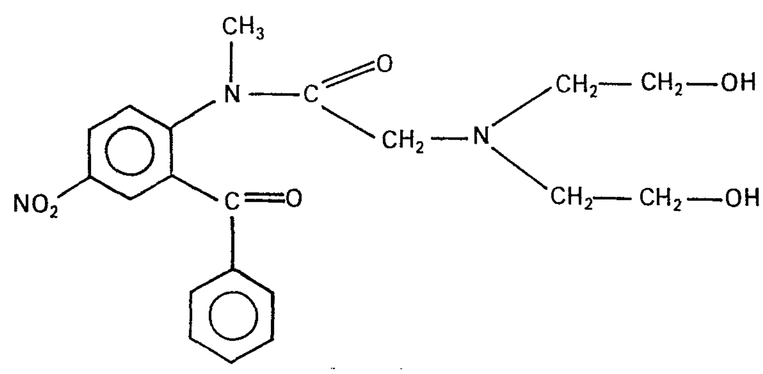

Exemple 17

N′-méthyl (méthyl-4′ pipérazino)-2 (benzoyl-2 nitro-4) acétanilide

D'une manière analogue à celle décrite dans l'exemple 10, mais en utilisant la N-méthyl pipérazine, on obtient le produit de formule:

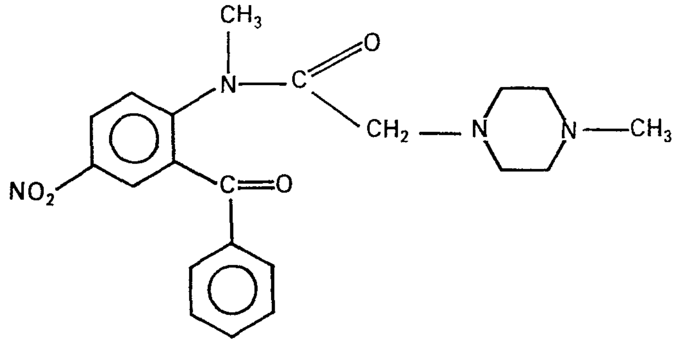

Les composés de la présente invention, doués d'une remarquable activité sur le système nerveux central, sont donc susceptibles d'être administrés à l'homme ou à l'animal par voie orale ou par injection, sous la forme d'une base libre ou bien de l'un de ses sels thérapeutiquement acceptables.

A titre de simple illustration, on indiquera ci-après quelques résultats de divers essais toxicologiques et pharmacologiques effectués sur les composés chimiques de l'invention.

Propriétés pharmacologiques

A) Tests utilisés

1. Etude de la toxicité

Les composés de la présente invention ont été soumis à des contrôles de toxicités. La dose létale 50 est comprise entre 500 et 3000 mg/kg pour l'ensemble des dérivés testés. Elle a été recherchée par voie orale et calculée selon la méthode de Miller et Tainter (Proc. Soc. exper. Biol. Med., 1944, 57, 261).

2. Activité dans l'essai sur la tige tournante (Rota-Rod)

On réalise cet essai sur des souris mâles de souche Swiss. On place la souris sur une tige de bois d'un diamètre de 3 cm tournant à raison de 5 tours par minute. On choisit les souris qui peuvent rester sur la tige pendant au moins 3 minutes au cours d'essais successifs et on rassemble par groupe de 10 pour l'essai de chaque dose. Si la souris tombe de la tige en moins de deux minutes, on considère le composé essayé comme efficace.

Les résultats des composés sont exprimés en $DE_{50}$ selon: N.W. Dunham et T.S. Miva – J. Amer. Pharm. Asso., 1957, 46, 208. Les $DE_{50}$ des composés les plus intéressants varient de 0,2 à 20 mg/kg.

3. Activité antagoniste au pentétrazol

Ce test est réalisé sur un groupe de 10 souris mâles de souche Swiss. Dans un délai de 15 minutes après l'injection sous-cutanée de 125 mg/kg de pentétrazol, les souris ont des convulsions toniques dont l'issue est fatale. Pour l'essai, on administre par voie orale les composés 60 minutes avant l'injection de pentétrazol. Les animaux sont observés pendant 2 heures après administration du pentétrazol.

Les résultats sont exprimés par la dose efficace $DE_{50}$ selon Goodmann et coll. – J. Pharmacol. 108, 1953. Les $DE_{50}$ des composés les plus intéressants varient de 0,1 à 3 mg/kg.

Applications therapeutiques

Compte tenu de leurs propriétés pharmacologiques et de leur faible toxicité, ces composés chimiques peuvent être utilisés en thérapeutiques, et plus particulièrement pour le traitement des insomnies.

Ces composés et leurs sels d'addition d'acides thérapeutiquement compatibles peuvent être utilisés comme médicaments, par exemple sous forme de préparations pharmaceutiques adaptées à l'administration entérale ou parentérale, avec par exemple, l'eau, le lactose, la gélatine, les amidons, le stéarate de magnésium, le talc, les huiles végétales, les gommes, les polyalcoylèneglycols, la vaseline, etc.

Ces préparations peuvent se présenter sous forme solide, par exemple de comprimés, dragées, capsules, etc. ou sous forme liquide, par exemple de solutions, suspensions ou émulsions.

Les préparations pharmaceutiques sous une forme appropriée à l'injection sont préférées. Ces préparations peuvent être soumises à des opérations pharmaceutiques classiques telles que stérilisation et/ou peuvent contenir des adjuvants par exemple des agents conservateurs, stabilisants, de mouillage ou d'émulsification, des composés-tampons, etc.

Les dosages, auxquels les composés actifs et leurs sels d'addition d'acides thérapeutiquement compatibles peuvent être administrés, peuvent varier dans des proportions importantes selon l'état du patient. Un dosage quotidien d'environ 0,01 mg à 1 mg par kg de poids corporel est toutefois préféré.

Les compositions pharmaceutiques selon l'invention peuvent être utilisées en médecine, par exemple dans le traitement des insomnies. De telles compositions peuvent en outre contenir d'autres principes actifs venant compléter ou renforcer l'action thérapeutique des dérivés de formule générale I selon la présente invention.

Bien entendu, la présente invention ne se trouve pas limitée aux exemples particuliers mentionnés à simple titre illustratif, mais il est parfaitement possible, sans pour autant sortir du cadre de l'invention, d'en imaginer un certain nombre de variantes et de modifications.

**Revendications**

1. Nouveaux dérivés de benzoyl-2 nitro-4 anilides répondant à la formule générale (I):

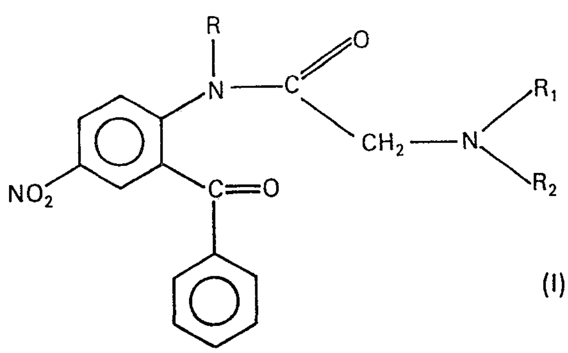

(I)

dans laquelle:

R représente un groupe alcoyle linéaire ou ramifié, un groupe alcényle, un groupe cycloalcoyle ou un groupe benzyle;

$R_1$ représente un atome d'hydrogène, un groupe alcoyle ou hydroxyalcoyle;

$R_2$ représente un groupe hydroxyalcoyle, alcényle ou alcynyle éventuellement substitués une ou plusieurs fois par un radical alcoyle, ou un groupe cycloalcoyle de 3 à 6 atomes de carbone;

$R_1$ et $R_2$ peuvent en outre former avec l'atome d'azote auquel ils sont fixés un hétérocycle azoté contenant éventuellement un second hétéroatome choisi parmi l'oxygène et l'azote;
à la condition toutefois que, lorsque $R_2$ représente un groupe hydroxyalcoyle, $R_1$ ne peut être un atome d'hydrogène,
ainsi que leurs sels obtenus avec des acides minéraux ou organiques thérapeutiquement acceptables.

2. Le chlorhydrate du N-méthyl benzoyl-2′ nitro-4′ morpholino-2 acétanilide selon la revendication 1.

3. Le maléate acide de N-éthyl benzoyl-2′ nitro-4′ morpholino-2 acétanilide selon la revendication 1.

4. Le maléate acice de N-propyl benzoyl-2′ nitro-4′ morpholino-2 acétanilide selon la revendication 1.

5. Le maléate acide de N-isopropyl benzoyl-2′ nitro-4′ morpholino-2 acétanilide selon la revendication 1.

6. Le maléate de N-allyl benzoyl-2′ nitro-4′ morpholino-2 acétanilide selon la revendication 1.

7. Le N-benzyl benzoyl-2′ nitro-4′ morpholino-2 acétanilide selon la revendication 1.

8. Le N-cyclopropyl benzoyl-2′ nitro-4′ morpholino-2 acétanilide selon la revendication 1.

9. Le N-cyclopentyl benzoyl-2′ nitro-4′ morpholino-2 acétanilide selon la revendication 1.

10. Le N-cyclohexylbenzoyl-2′ nitro-4′ morpholino-2 acétanilide selon la revendication 1.

11. Le chlorhydrate de N-cyclohexyl N′ méthyl benzoyl-2′ nitro-4′ glycylanilide selon la revendication 1.

12. Le fumarate de N-N′ diméthyl N-cyclohexyl benzoyl-2′ nitro-4′ glycylanilide selon la revendication 1.

13. Le N′-méthyl N cyclopropyl benzoyl-2′ nitro-4′ glycylanilide selon la revendication 1.

14. Le N′-méthyl N diméthyl-1-1 propargyl benzoyl-2′ nitro-4′ glycylanilide selon la revendication 1.

15. Le N′-méthyl N allyl benzoyl-2′ nitro-4′ glycylanilide selon la revendication 1.

16. Le N-N′ diméthyl N (hydroxy-2 éthyl) benzoyl-2′ nitro-4′ glycylanilide selon la revendication 1.

17. Le N-méthyl N-N bis (hydroxy-2 éthyl) benzoyl-2′ nitro-4′ glycylanilide selon la revendication 1.

18. Le N-méthyl méthyl-4′ pipérazino-2 (benzoyl-2 nitro-4) acétanilide selon la revendication 1.

19. Procédé de préparation des dérivés de formule générale I selon l'une des revendications 1 à 18, caractérisé par le fait qu'il consiste à faire réagir un halo-acétamide de formule générale II

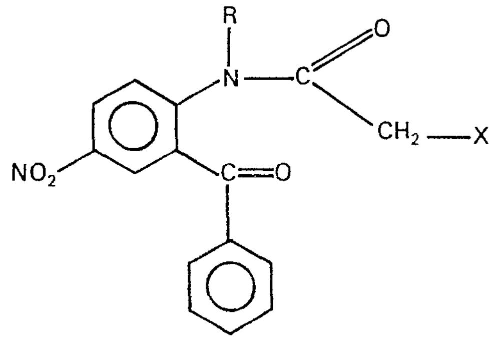

(II)

dans laquelle:
R a la signification donnée à la revendication 1 et X représente un atome d'halogène,
avec une amine de formule III:

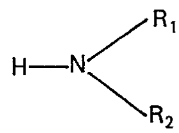

(III)

dans laquelle:
$R_1$ et $R_2$ ont les significations données à la revendication 1.

20. A titre de médicaments nouveaux, les dérivés de formule générale I selon l'une des revendications 1 à 18.

21. Les compositions pharmaceutiques contenant comme principe actif au moins un dérivé de formule générale I selon l'une des revendications 1 à 18.

22. Les compositions pharmaceutiques selon la revendication 21 dans lesquelles aux produits selon l'une des revendications 1 à 18, peuvent être associés d'autres principes actifs venant compléter ou renforcer leurs actions thérapeutiques.

**Claims**

1. New derivatives of 2-benzoyl-4-nitro-anilides corresponding to the following general formula:

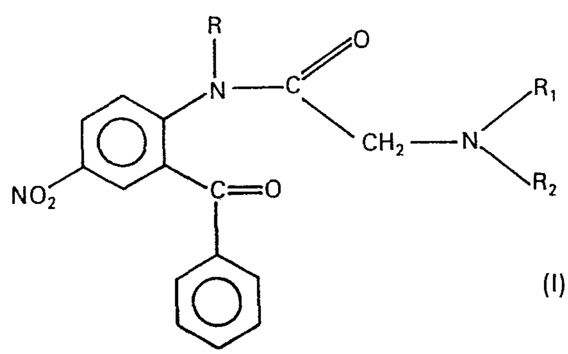

(I)

in which
R represents a linear or branched alkyl group, an alkenyl group, a cycloalkyl group or a benzyl group;

$R_1$ represents a hydrogen atom, an alkyl or hydroxy alkyl group;

$R_2$ represents a hydroxy alkyl, alkenyl or alkynyl group optionally substituted once or several times by an alkyl radical or a cycloalkyl group containing from 3 to 6 carbon atoms;

$R_1$ and $R_2$ may in addition form with the nitrogen atom to which they are attached a nitrogen-containing heterocycle optionally containing a second heteroatom selected from oxygen and nitrogen;

with the proviso that, where $R_2$ is a hydroxy alkyl group, $R_1$ cannot be a hydrogen atom, and their salts obtained with therapeutically acceptable mineral or organic acids.

2. N-methyl-2'-benzoyl-4'-nitro-2-morpholino-acetanilide hydrochloride according to claim 1.

3. The acid maleate of N-ethyl-2'-benzoyl-4'nitro-2-morpholino-acetanilide according to claim 1.

4. The acid maleate of N-propyl-2'-benzoyl-4'nitro-2-morpholino-acetanilide according to claim 1.

5. The acid maleate of N-isopropyl-2'-benzoyl-4'-nitro-2-morpholino-acetanilide according to claim 1.

6. The maleate of N-allyl-2'-benzoyl-4'nitro-2-morpholino-acetanilide according to claim 1.

7. N-benzyl-2'-benzoyl-4'-nitro-2-morpholino-acetanilide according to claim 1.

8. N-cyclopropyl-2'-benzoyl-4'-nitro-2-morpholino-acetanilide according to claim 1.

9. N-cyclopentyl-2'-benzoyl-4'-nitro-2-morpholino-acetanilide according to claim 1.

10. N-cyclohexyl-2'-benzoyl-4'-nitro-2-morpholino-acetanilide according to claim 1.

11. N-cyclohexyl-N'-methyl-2'-benzoyl-4'-nitro-glycylanilide hydrochloride according to claim 1.

12. N,N'-dimethyl-N-cyclohexyl-2'-benzoyl-4'-nitro-glycylanilide fumarate according to claim 1.

13. N'-methyl-N-cyclopropyl-2'-benzoyl-4'-nitro-glycylanilide according to claim 1.

14. N'-methyl-N-1,1-dimethyl-propargyl-2'-benzoyl-4'-nitro-glycylanilide according to claim 1.

15. N'-methyl-N-allyl-2'-benzoyl-4'-nitro-glycylanilide according to claim 1.

16. N,N'-dimethyl-N-(2-hydroxyethyl)-2'-benzoyl-4'-nitro-glycylanilide according to claim 1.

17. N'-methyl-N,N-bis-(2-hydroxyethyl)-2'-benzoyl-4'-nitro-glycylanilide according to claim 1.

18. N-methyl-4'-methyl-2-piperazino-(2-benzoyl-4-nitro)-acetanilide according to claim 1.

19. A process for preparing the derivatives corresponding to general formula I claimed in any of claims 1 to 18, characterised in that it comprises reacting a haloacetamide corresponding to the following general formula:

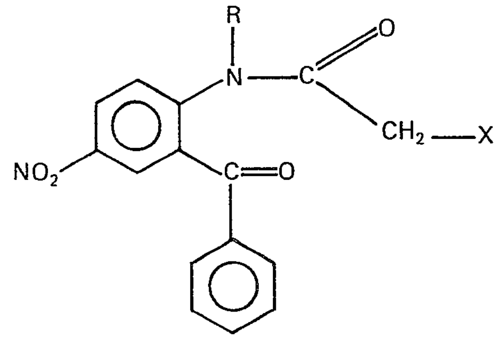

(II)

in which
R is as defined in claim 1 and
X represents a halogen atom,
with an amine corresponding to the following formula

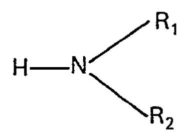

(III)

in which
$R_1$ and $R_2$ are as defined in claim 1.

20. As new medicaments, the derivatives corresponding to general formula I claimed in any of claims 1 to 18.

21. Pharmaceutical compositions containing as active principle at least one of the derivatives corresponding to general formula I claimed in any of claims 1 to 18.

22. Pharmaceutical compositions as claimed in claim 21, in which other active principles may be associated with the products claimed in any of claims 1 to 18 in order to complete or strengthen their therapeutic effects.

**Patentansprüche**

1. Neue 2-Benzoyl-4-nitroanilidderivate der allgemeinen Formel (I)

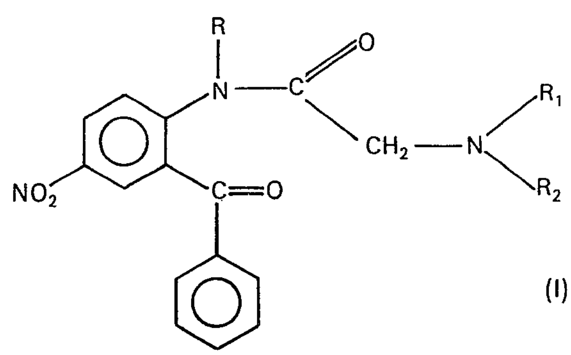

(I)

in welcher R eine lineare oder verzweigte Alkylgruppe, eine Alkenylgruppe, eine Cycloalkylgruppe oder eine Benzylgruppe darstellt;

$R_1$ Wasserstoff, eine Alkyl- oder Hydroxyalkylgruppe bedeutet;
$R_2$ eine Hydroxyalkylgruppe, eine Alkenyl- oder Alkinylgruppe, gegebenenfalls ein- oder mehrfach durch einen Alkylrest substituiert, oder eine Cycloalkylgruppe mit 3 bis 6 C-Atomen darstellt;
$R_1$ und $R_2$ aber auch mit dem Stickstoffatom, an das sie gebunden sind, einen stickstoffhaltigen Heterocyclus bilden können, der gegebenenfalls ein zweites Heteroatom ausgewählt unter Sauerstoff und Stickstoff enthält,
mit der Massgabe, dass, wenn $R_2$ eine Hydroxyalkylgruppe darstellt, $R_1$ kein Wasserstoffatom sein kann,
sowie deren mit Mineralsäuren oder organischen, therapeutisch annehmbaren Säuren erhaltene Salze.

2. Das Chlorhydrat von N-Methyl-2′-benzoyl-4′-nitro-2-morpholino-acetanilid gemäss Anspruch 1.

3. Das saure Maleat von N-Äthyl-2′-benzoyl-4′-nitro-2-morpholino-acetanilid gemäss Anspruch 1.

4. Das saure Maleat von N-Propyl-2′-benzoyl-4′-nitro-2-morpholino-acetanilid gemäss Anspruch 1.

5. Das saure Maleat von N-Isopropyl-2′-benzoyl-4′-nitro-2-morpholino-acetanilid gemäss Anspruch 1.

6. Das Maleat von N-Allyl-2′-benzoyl-4′-nitro-2-morpholino-acetanilid gemäss Anspruch 1.

7. Das N-Benzyl-2′-benzoyl-4′-nitro-2-morpholino-acetanilid gemäss Anspruch 1.

8. Das N-Cyclopropyl-2′-benzoyl-4′-nitro-2-morpholino-acetanilid gemäss Anspruch 1.

9. Das N-Cyclopentyl-2′-benzoyl-4′-nitro-2-morpholino-acetanilid gemäss Anspruch 1.

10. Das N-Cyclohexyl-2′-benzoyl-4′-nitro-2-morpholino-acetanilid gemäss Anspruch 1.

11. Das Chlorhydrat von N-Cyclohexyl-N′-methyl-2′-benzoyl-4′-nitro-glycinanilid gemäss Anspruch 1.

12. Das Fumarat von N,N′-Dimethyl-N-cyclohexyl-2′-benzoyl-4′-nitro-glycinanilid gemäss Anspruch 1.

13. Das N′-Methyl-N-cyclopropyl-2′-benzoyl-4′-nitro-glycinanilid gemäss Anspruch 1.

14. Das N′-Methyl-N-1,1-dimethyl-propargyl-2′-benzoyl-4′-nitro-glycinanilid gemäss Anspruch 1.

15. Das N′-Methyl-N-allyl-2′-benzoyl-4′-nitro-glycinanilid gemäss Anspruch 1.

16. Das N,N′-Dimethyl-N-(2-hydroxyäthyl)-2′-benzoyl-4′-nitro-glycinanilid gemäss Anspruch 1.

17. Das N′-Methyl-N,N-bis-(2-hydroxyäthyl)-2′-benzoyl-4′-nitro-glycinanilid gemäss Anspruch 1.

18. Das N-Methyl-4′-methyl-2-piperazino-(2-benzoyl-4-nitro)-acetanilid gemäss Anspruch 1.

19. Verfahren zur Herstellung von Derivaten der allgemeinen Formel I gemäss einem der Ansprüche 1 bis 18, dadurch gekennzeichnet, dass man ein Halogenacetamid der allgemeinen Formel II

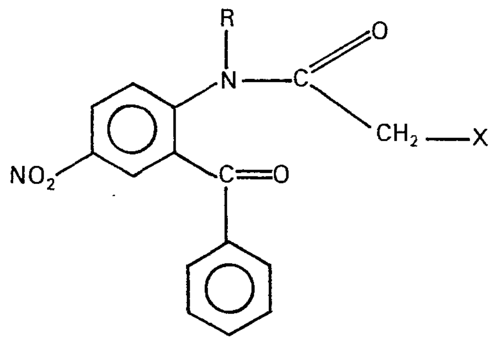
(II)

in welcher R die in Anspruch 1 angegebene Bedeutung hat und X ein Halogenatom darstellt, mit einem Amin der Formel III

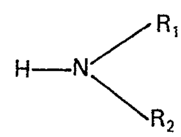
(III)

in welcher $R_1$ und $R_2$ die in Anspruch 1 angegebene Bedeutung haben, reagieren lässt.

20. Die Derivate der allgemeinen Formel I gemäss einem der Ansprüche 1 bis 18 als neue Heilmittel.

21. Die pharmazeutischen Zusammensetzungen, die als Wirkstoff wenigstens ein Derivat der allgemeinen Formel I gemäss einem der Ansprüche 1 bis 18 enthalten.

22. Die pharmazeutischen Zusammensetzungen gemäss Anspruch 21, in welchen die Produkte gemäss einem der Ansprüche 1 bis 18 mit anderen Wirkstoffen assoziiert sein können, was zu einer Komplettierung oder Verstärkung ihrer therapeutischen Wirkungen führt.